# EUROPÄISCHE PATENTSCHRIFT

(11) **EP 0 834 318 B1**
(45) Veröffentlichungstag und Bekanntmachung des Hinweises auf die Patenterteilung: **08.08.2001**
(21) Anmeldenummer: 97117041.0
(22) Anmeldetag: 01.10.1997
(51) Int. Cl.: A61K 33/24, A61K 31/70, A61K 31/715

(54) **Chromhaltiges Mittel zur Steuerung der cellulären Glucoseaufnahme**
Composition comprising chromium for controlling cellular glucose uptake
Composition comprenant du chrome pour réguler l'absorption cellulaire du glucose

(30) Priorität: 01.10.1996 DE 19640602
(43) Veröffentlichungstag der Anmeldung: 08.04.1998
(73) Patentinhaber: Dölcken, Carola, 47447 Moers (DE)
(72) Erfinder: Dölcken, Carola, 47447 Moers (DE)
(74) Vertreter: Blumbach, Kramer & Partner GbR

(56) Entgegenhaltungen:
- EP-A- 0 037 144
- EP-A- 0 265 772
- DE-A- 2 330 069
- US-A- 5 614 224
- NIELSEN F H : "CONTROVERSAL CHROMIUM. DOES THE SUPERSTAR MINERAL OF THE MOUNTEBANKS RECEIVE APPROPRIATE ATTENTION FROM CLINICIANS AND NUTRITIONISTS?" NUTRITION TODAY, Bd. 31, Nr. 6, November 1996, USA, Seiten 226-233, XP002053838
- PATENT ABSTRACTS OF JAPAN vol. 009, no. 180 (C-293), 25.Juli 1985 & JP 60 048993 A (TERUMO KK), 16.März 1985,

## Beschreibung

Die vorliegende Erfindung betrifft ein chromhaltiges Mittel, mit dem die Verwertung von Glucose im Blut durch die Zellen gesteuert werden kann.

Glucose als in freier oder in zahlreichen Molekülen (einschließlich Dimeren, Oligomeren und Polymeren) gebundener Form vorkommendes Monosaccharid wird in Pflanzen durch Photosynthese erzeugt und kann daher als für die menschliche Nahrung in großen Mengen zur Verfügung stehende, regenerierbare Energiequelle angesehen werden. Im menschlichen Körper werden Glucose oder Glucose enthaltende Moleküle (z. B. Disaccharide, Oligosaccharide, Polysaccharide) im Rahmen der Glycolyse unter Energiegewinn abgebaut ("verstoffwechselt"). Glucose ist damit für den menschlichen Körper eine Hauptenergiequelle. Insbesondere für die Hirn- und Nervenzellen gilt Glucose als der wichtigste, schnell verfügbare (und auch die Blut-Hirn-Schranke passierende) Energieträger.

Durch Nahrungsaufnahme und Resorption durch die Wände des Magen-Darm-Traktes werden die Nahrungs-Inhaltsstoffe einschließlich Glucose und ihrer Vorstufen in das Blut überführt. Damit die Konzentration der Glucose im Blut keinen Schwankungen unterliegt und damit die lebenswichtige kontinuierliche Versorgung der Zellen, insbesondere der Gehirnzellen, mit Glucose gesichert ist, wird durch ein hormonell (beispielsweise durch Adrenalin oder Glucagon) gesteuertes Gleichgewicht bei Glucosemangel als "Vorrat" aufgebautes Glykogen (Polymer der Glucose) der Leber abgebaut und die resultierende Glucose dem Blutkreislauf zugeführt, während bei Glucoseüberschuß (z. B. durch Nahrungsaufnahme) Glucose der Leber zum Aufbau von Glykogen zugeführt wird. Durch dieses Gleichgewicht wird die Glucosekonzentration im Blut auf einem im wesentlichen konstanten Wert von etwa 720 bis 900 mg/l Blut gehalten.

Ein kontinuierlicher Glucosefluß aus dem Blut in die einzelnen Zellen durch Einschleusen der hydrophilen Glucosemoleküle durch die lipophile Zellmembran mittels Transporter-Molekülen. Das Einwandern durch die Zellmembran wird durch Änderungen der Permeabilität der Zellmembran erreicht. Die Feinsteuerung der Permeabilität leisten sogenannte "Glucosetoleranzfaktor-Rezeptoren". Diese benötigen Cr³⁺-Ionen als Cofaktoren.

Diese bekannte Tatsache hat dazu geführt, daß versucht wurde, über die Ernährung die Cr-Werte im Blut auf einen Wert zu bringen, der die Feinsteuerung der Permeabilität der Zellmembran durch die Glucosetoleranzfaktor-Rezeptoren ermöglicht. Bisher war dies jedoch nur unzureichend möglich. Es bestand daher ein Bedarf danach, und die Erfindung hat sich zur Aufgabe gestellt, ein Mittel zur Verfügung zu stellen, mit dem eine langzeitgesteuerte Versorgung der Zellen mit Energie durch Glucosezufuhr gesichert werden kann. Insbesondere war es eine Aufgabe der vorliegenden Erfindung, eine kontinuierliche Glucosezufuhr zur Zelle unter Vermeidung der durch Glucosemangel in der Zelle bedingten Leistungsabfälle zu realisieren.

Überraschend wurden diese Aufgaben gelöst durch oral verabreichbare Mittel, die neben Glucose Chrom in für die Feinsteuerung der Glucosetoleranzfaktor-Rezeptoren geeigneter Konzentration enthalten.

Die Erfindung betrifft eine Zubereitung, die Glucose und/oder eine oder mehrere unter physiologischen Bedingungen Glucose abgebende Verbindung(en) und Chrom in resorbierbarer Form sowie einen Emulgator/Wirkstoff neben physiologisch annehmbaren Träger- und Hilfsstoffen enthält, worin
- Glucose und/oder eine oder mehrere unter physiologischen Bedingungen Glucose abgebende Verbindung(en) in einer Konzentration von 30 bis 60 Gew.-%;
- Chrom in einer Konzentration von 0,002 bis 0,01 Gew.-%; und
- ein oder mehrere Emulgator(en)/Wirkstoff(e) in einer Gesamtkonzentration von 0,5 bis 2 Gew.-% enthalten sind.

In einer alternativen Ausführungsform der Erfindung ist in der Zubereitung anstelle des Emulgators/Wirkstoffs oder zusammen mit diesem Cholinbitartrat in einer Konzentration im Bereich von 0,05 bis 0,5 Gew.-% enthalten, wie in Patentanspruch 2 beansprucht ist.

Bevorzugte Zubereitungen ergeben sich aus den Unteransprüchen 3 bis 6.

Erfindungsgemäß können solche Zubereitungen dem Körper auf verschiedenen Wegen zugeführt werden. Bevorzugt sind dabei die Verabreichung der erfindungsgemäßen Zubereitungen auf oralem Weg, da diese Darreichungsform keiner besonderen Fachkunde bedarf und eine sachgerechte Dosierung auch jedem Laien möglich ist. In besonderen Fällen, beispielsweise dann, wenn eine schnelle und effiziente Energiezufuhr für den Körper lebensnotwendig ist, kann jedoch auch eine intravenöse Verabreichung einer erfindungsgemäßen Zubereitung erfolgen. Dies führt die Energie liefernden Moleküle wie auch die notwendigen Cr-Ionen direkt zum Wirkort. Die Verabreichung ist jedoch nicht auf diese beiden Wege beschränkt.

Erfindungsgemäß können die Zubereitungen gegebenenfalls auch weitere Hilfsstoffe enthalten. Derartige Zubereitungen ergeben sich ebenfalls aus Patentanspruch 6.

Die erfindungsgemäßen Zubereitungen können für medizinische Zwecke vorgesehen werden. Dies ist insbesondere dann der Fall, wenn aus irgendwelchen Gründen eine Situation gegeben ist, in der dem Körper schnell verwertbare Glucose zugeführt werden muß, wie beispielsweise nach Operationen (künstliche Ernährung), in der Rekonvalenszenz-Zeit oder in solchen Lebensphasen, in denen der Körper auf eine effiziente Zufuhr von schnell Energie liefernden Nährstoffen angewiesen ist. Dies schließt die arzneimittel-wirksame Zufuhr der erfindungsgemäßen Zubereitungen ein.

Andererseits ist es auch möglich, die erfindungsgemäßen Zubereitungen aus ernährungsphysiologischen Gründen zuzuführen. Dies schließt beispielsweise den Fall ein, in dem es erforderlich ist, Glucose enthaltende Nahrungsergänzungsmittel zuzusetzen, beispielsweise in Entwicklungsländern, in denen die wenig effiziente Energieverwertung im Ernährungsbereich oft zu Mangelzuständen führt. Diese können mit den erfindungsgemäßen Zubereitungen in vergleichsweise kurzer Zeit behoben werden. Insbesondere ist es in solchen Fällen möglich, dem Körper ein ausgewogenes "Mix" an ernährungsphysiologisch wertvollen Stoffen zuzuführen, was eine synergistisch fördernde Wirkung auf den physiologischen Gesamtzustand hat. Umfaßt von der Zufuhr der erfindungsgemäßen Zubereitungen aus ernährungsphysiologischen Gründen ist auch die Verabreichung an Leistungssportler. Diese benötigen insbesondere in Wettkampfsituationen auch schnelle Energiespender unter Verbesserung der Glucose-Resorption. Mit den erfindungsgemäßen Zubereitungen läßt sich das (leistungsbestimmende) Verhältnis Kohlendioxid/Sauerstoff im Blut zugunsten des Sauerstoffs verbessern und macht damit eine Steigerung der Ausdauerleistung und/oder eine schnellere Regeneration möglich.

Weiter kann auch an eine diabetische Verwendung der erfindungsgemäßen Zubereitungen gedacht werden, beispielsweise bei Unterzuckerung (Hypoglykämie) infolge zu starker Insulinzufuhr bei Diabetikern oder bei Unterzuckerung infolge erzwungenen oder freiwilligen Verzichts auf Glucose oder deren Vorstufen enthaltende Nahrung.

In bevorzugten Ausführungsformen der erfindungsgemäßen Zubereitungen ist Glucose und/oder eine oder mehrere unter physiologischen Bedingungen Glucose abgebende Verbindung(en) in einer Konzentration von 35 bis 50 Gew.-% enthalten. Diese Konzentration ist besonders dann von Vorteil, wenn die Zubereitung auch noch andere Stoffe, beispielsweise Hilfsstoffe für die Darreichung, in nennenswerten Mengen enthält. Die Konzentrationsangaben sind jedoch nicht auf die vorstehend angegebenen Bereiche beschränkt.

Es entspricht einer weiteren bevorzugten Ausführungsform der Erfindung, wenn Chrom, insbesondere in Form von Cr³⁺-Ionen, in einer Konzentration im Bereich von 0,004 bis 0,008 Gew.-% enthalten ist. Mit Zubereitungen mit Cr-Gehalten in diesem Bereich läßt sich in besonders einfacher Weise eine Feinsteuerung der Glucose-Aufnahme erreichen.

Weiter ist es erfindungsgemäß bevorzugt, daß die Zubereitungen einen oder mehrere Emulgator(en)/Wirkstoff(e) in Konzentrationen von 0,8 bis 1,8 Gew.-% enthalten. Dies ist insbesondere dann von Vorteil, wenn es die Darreichungsform erfordert, daß die Zubereitung fein emulgiert ist, ohne daß es zu Ausfällungen aus der flüssigen Phase kommt.

In besonders bevorzugten Ausführungsformen der erfindungsgemäßen Zubereitungen sind die als Haupt-Bestandteil verwendete Glucose und die unter physiologischen Bedingungen Glucose abgebenden Verbindungen gewählt aus D-Glucose, L-Glucose, D- oder L-Glucose enthaltenden Disacchariden wie Saccharose, Maltose oder Lactose, D- oder L-Glucose enthaltenden Polysacchariden wie Stärke oder Glykogen. Von den genannten Verbindungen können alle einzeln oder in beliebigen Mischungen von zwei oder mehreren der genannten Verbindungen verwendet werden. Mit besonderem Vorteil wird D-Glucose und/oder solche Verbindungen als Komponente der erfindungsgemäßen Zubereitungen verwendet, die unter physiologischen Bedingungen D-Glucose abspalten, die dieses Isomer der Glucose dasjenige ist, das physiologisch optimal verwertet werden kann.

In den erfindungsgemäßen Zubereitungen ist es bevorzugt, daß das Chrom in resorbierbarer und physiologisch verträglicher Form zugegen ist. Eine solche Form ist Chrom in Form von Cr³⁺-Ionen, wie sie beispielsweise in physiologisch verträglichen Chrom(III)-Salzen vorhanden sind. Erfindungsgemäß wird mit besonderem Vorteil Chrom(III)-Picolinat verwendet; dieses Salz ist physiologisch ausgezeichnet verträglich und ist darüber hinaus zur Verwendung in Lebensmitteln und Arzneimitteln zugelassen. Weiter ist es erfindungsgemäß möglich, Chrom in Form von Chromhefe, Bierhefe mit einem natürlichen Chrom-Gehalt oder anderen Materialien natürlicher Herkunft einzusetzen, wie sie auf dem Markt beispielsweise unter dem Handelsnamen Levurinetten^{R} (Fa. Zyma) oder Bierhefe (Fa. Biolabor) erhältlich sind. Hierbei sind dann die Konzentrationen dieses Chrom-"Lieferanten" so anzusetzen, wie es den erfindungsgemäßen Chrom-Konzentrationen entspricht.

Als Emulgator/Wirkstoff können die erfindungsgemäßen Zubereitungen jeden Emulgator/ Wirkstoff enthalten, der lebensmittel- und arzneimittelrechtlich zugelassen ist, physiologisch verträglich ist und mit den anderen Komponenten keine unerwünschten Reaktionen eingeht. Besonders bevorzugt ist als Emulgator Lecithin, weil es aus natürlichen Quellen erhältlich ist und dem Körper auch aus anderen Quellen "bekannt" ist. In einer besonders bevorzugten Ausführungsform der Erfindung wird als Emulgator Sojabohnen-Lecithin verwendet, wobei es sich ganz besonders bewährt hat, Sojabohnen-Lecithin zu verwenden, das einen hohen Phosphatidyl-Anteil aufweist. Mit diesem besonderen Sojabohnen-Lecithin, das beispielsweise unter der Bezeichnung Nathin SM 100 ^{R} im Handel erhältlich ist, kann nämlich der sonst mitunter als unangenehme Geschmack empfundene des Lecithins vermieden werden, was zu einer ausgezeichneten Akzeptanz der erfindungsgemäßen Zubereitung bei der Einnahme führt.

Einer weiteren erfindungsgemäß bevorzugten Ausführungsform kann der vorstehend genannte Emulgator vollständig oder teilweise ersetzt sein durch Cholinbitartrat. Dieses wird zugesetzt in solchen Mengen, daß die erwünschte Wirkung einer Emulgierung zur guten Verabreichbarkeit erreicht wird. Erfindungsgemäß liegt die Konzentration an Cholinbitartrat im Bereich von 0,05 bis 0,5 Gew.-%, bevorzugt bei 0,1 bis 0,3 Gew.-%, besonders bevorzugt bei 0,15 bis 0,2 Gew.-%. Wie bereits festgestellt, kann Cholinbitartrat den Emulgator ganz oder teilweise ersetzen, d. h. es kann zusammen mit diesem oder anstelle des Emulgators in den erfindungsgemäßen Zubereitungen enthalten sein.

Wenn dies auch nicht essentiell ist, können die erfindungsgemäßen Zubereitungen - wie oben bereits angegeben - zusätzlich physiologisch annehmbare weitere Stoffe wie beispielsweise Vitamine und Spurenelemente und gegebenenfalls auch Träger- und Hilfsstoffe wie den pH-Wert einstellende Mittel, Stabilisatoren, Puffer, Salze, Lösemittel, Geschmacksstoffe, Geruchsstoffe, das Lösen in Lösemitteln fördernde Mittel, durch die jeweilige Verabreichungs- bzw. Darreichungsform bedingte Hilfsstoffe sowie auch andere Stoffe enthalten. Beispielsweise sind Vitamine wie Vitamine der B-Reihe, Vitamin C und/oder Folsäure in solchen Mengen zugegen, daß die üblichen Tagesbedarf-Mengen des menschlichen Körpers damit zugeführt werden können. Unterschiedliche Dosierungen für Menschen unterschiedlichen Alters, unterschiedlichen Entwicklungsstadiums, unterschiedlicher Lebensweise u. ä. sind dabei selbstverständlich möglich. In gleicher Weise können neben Chrom auch andere Spurenelemente wie beispielsweise Magnesium, Natrium, Kalium, Eisen etc. in beliebiger Kombination in Mengen zugesetzt sein, daß dies ebenfalls den zuzuführenden Tagesbedarf-Mengen entspricht.

Wenn die Verabreichungsform dies erfordert, können auch Lösemittel wie Wasser, wäßrige (gegebenenfalls Salze oder andere Stoffe enthaltende) Lösungen in den Zubereitungen gemäß der Erfindung enthalten sein. So können Lösungen zur Einnahme oder sogar intravenösen Verabreichung gebildet werden.

In bevorzugten Ausführungsformen der Erfindung liegen die Zubereitungen in Form von Pulvern vor. Diese können entweder der üblichen Nahrung zugesetzt oder auch in einem geeigneten Lösemittel (wie z. B. Wasser) aufgeschlämmt, emulgiert oder gelöst und dann oral eingenommen werden. In gleicher Weise können die Zubereitungen auch in Form von Granulaten, Brausetabletten, Dragees, Tabletten, Kapseln wie beispielsweise mit den pulverförmigen oder in Granulatform überführten Zubereitungen gefüllten Weichgelatinekapseln, Pasten, Lösungen zur Infusion oder oralen Aufnahme und anderen, dem Fachmann an sich bekannten Verabreichungsformen konfektioniert werden. Seitens der Zubereitungen und ihrer Inhaltsstoffe unterliegt die Darreichungsform keinen Beschränkungen.

Die Erfindung wird weiter unter Bezugnahme auf nachfolgende Rezepturbeispiele einzelner Zubereitungen näher erläutert, ohne auf diese beschränkt sein.

### Rezepturbeispiele 1 bis 4

Es wurden ein chromhaltige Mittel zur Förderung der cellulären Glucoseaufnahme aus folgenden Komponenten formuliert:

| **Komponenten (Mengen in g)** | **Bsp. 1** | **Bsp. 2** | **Bsp. 3** | **Bsp. 4** |
|---|---|---|---|---|
| D-Glucose | 3,6 | 3,0 | 2,6 | 2,0 |
| Fructose | - | 0,6 | 1,0 | 1,6 |
| Chrom(III)-Picolinat (g x 10⁻⁶) | 250 | 250 | 250 | 250 |
| Lecithin-Konzentrat | 1,2 | 1,5 | 1,2 | 0,9 |
| Cholinbitartrat | - | - | 0,3 | 0,6 |
| Magnesium | 0,900 | 0,900 | 0,800 | 0,850 |
| Vitamin C | 0,200 | 0,225 | 0,200 | 0,225 |
| Folsäure (g x 10⁻³) | 0,3 | 0,4 | 0,4 | 0,3 |
| Vitamin B-Komplex ¹⁾ (g x 10⁻³) | 10 | 15 | 10 | 15 |

| | | | | |
|---|---|---|---|---|
| Anmerkung 1): Der Vitamin B-Komplex enthielt Vitamin B1, Vitamin B2, Vitamin B6 und Vitamin B12 im Mengenverhältnis 4 : 4 : 2 : 4 x 10⁻³. | | | | |

Die genannten Komponenten wurden intensiv gemischt und ergaben jeweils ein frei fließendes Pulver. Dieses wurde als solches in Dosen gefüllt. Der Doseninhalt betrug beispielsweise 250 g, 180 g o. ä..

In gleicher Weise wurde das erhaltene Pulver in Briefchen aus einem innen beschichteten, wasserundurchlässigen Papier gefüllt, beispielsweise in Mengen von 5, 6 oder 8 g (= 1 Tagesdosis).

Die jeweiligen Pulver konnten zusammen mit der üblichen festen Nahrung (unter Darüberstreuen) oder in Wasser aufgelöst oral eingenommen werden.

### Rezepturbeispiele 5 bis 8

Die nach den Beispielen 1 bis 4 erhaltenen Pulver wurden zusammen mit Natriumcarbonat und einem üblichen Hilfsstoff für die Beibehaltung der Komprettenform, beispielsweise mit Lactose oder Natriumstearat (wobei ersteres den Vorteil aufweist, auch als Glucose freisetzender Bestandteil der Zubereitung zu dienen), sorgfältig vermischt, wobei die Mengenverhältnisse pulverförmige Zubereitung : Natriumcarbonat : Lactose (bzw. in Beispiel 8: Natriumstearat) wie 1 : 0,5 : 1 waren. Die erhaltenen Pulver wurden auf ausreichend dimensionierten Pulverpressen zu Brausetabletten verpreßt. Diese lösten sich ausgezeichnet in Wasser und konnten so zu einem schmackhaften Getränk für die orale Verabreichung zubereitet werden.

### Rezepturbeispiele 9 bis 16

In den Zubereitungen gemäß den Rezepturbeispielen 1 bis 4 wurde Chrom(III)-Picolinat durch Chromhefe (Beispiele 9 bis 12) bzw. chromhaltige Bierhefe (Beispiele 13 bis 16) in je einer solchen Menge ersetzt, daß die Chromkonzentration gleich derjenigen war, wie sie durch die Verwendung von Chrom(III)-Picolinat in den Beispielen 1 bis 4 eingestellt worden war.

Es wurden vergleichbare Zubereitungen erhalten, die im wesentlichen dieselben Eigenschaften hatten wie die Zubereitungen nach den Beispielen 1 bis 4, wobei die chromhaltige Bierhefe am besten vertragen wurde.

### Rezepturbeispiele 17 bis 20

In den Rezepturen der Beispiele 1 bis 4 wurde die Glucose zu 50 % der Gewichtsmenge durch Lactose ersetzt. Es wurden erfindungsgemäße Zubereitungen erhalten, die in gleicher Weise vertragen wurden und wirkten wie die Zubereitungen der Rezepturbeispiele 1 bis 4 und die insbesondere für Kleinkinder mit Vorteil eingesetzt werden konnten.

## Patentansprüche

1. Zubereitung, enthaltend Glucose und/oder eine oder mehrere unter physiologischen Bedingungen Glucose abgebende Verbindung(en) und Chrom in resorbierbarer Form sowie einen Emulgator/Wirkstoff neben physiologisch annehmbaren Träger- und Hilfsstoffen, worin
- Glucose und/oder eine oder mehrere unter physiologischen Bedingungen Glucose abgebende Verbindung(en) in einer Konzentration von 30 bis 60 Gew.-%;
- Chrom in einer Konzentration von 0,002 bis 0,01 Gew.-%; und
- ein oder mehrere Emulgator(en)/Wirkstoff(e) in einer Gesamtkonzentration von 0,5 bis 2 Gew.-% enthalten sind.

2. Zubereitung nach Anspruch 1, enthaltend anstelle von oder zusammen mit einem oder mehreren Emulgator(en) Cholinbitartrat in einer Konzentration von 0,05 bis 0,5 Gew.-%, bevorzugt in einer Konzentration von 0,1 bis 0,3, besonders bevorzugt von 0,15 bis 0,2 Gew.-%.

3. Zubereitung nach Anspruch 1 oder Anspruch 2, worin Glucose und/oder eine oder mehrere unter physiologischen Bedingungen Glucose abgebende Verbindung(en) in einer Konzentration von 35 bis 50 Gew.-% enthalten ist/sind, bevorzugt worin als Glucose und unter physiologischen Bedingungen Glucose abgebende Verbindungen D-Glucose, L-Glucose, D- oder L-Glucose enthaltende Disaccharide wie Saccharose, Maltose oder Lactose, D- oder L-Glucose enthaltende Polysaccharide wie Stärke oder Glykogen und Mischungen der genannten Verbindungen in einer Konzentration von 35 bis 50 Gew.-% enthalten ist/sind.

4. Zubereitung nach einem oder mehreren der Ansprüche 1 bis 3, worin Chrom in einer Konzentration von 0,004 bis 0,008 Gew.-% enthalten ist, bevorzugt worin Chrom in Form von Chrom(III)-Picolinat, Chromhefe und/oder Bierhefe mit natürlichem Chromgehalt in einer Konzentration von 0,004 bis 0,008 Gew.-% enthalten ist/sind.

5. Zubereitung nach einem der Ansprüche 1 bis 4, worin ein oder mehrere Emulgator(en) in einer Gesamtkonzentration von 0,8 bis 1,8 Gew.-% enthalten ist/sind, bevorzugt worin Lecithin als Emulgator in einer Konzentration von 0,8 bis 1,8 Gew.-% enthalten ist, noch mehr bevorzugt worin Lecithin aus Soja-Lecithinen, insbesondere solchen mit hohem Phosphatidyl-Anteil, als Emulgator in einer Konzentration von 0,8 bis 1,8 Gew.-% enthalten ist.

6. Zubereitung nach einem der Ansprüche 1 bis 5, enthaltend zusätzlich Vitamine und Spurenelemente in physiologisch annehmbaren Konzentrationen sowie gegebenenfalls stattdessen oder zusätzlich Hilfsstoffe wie Geschmacksstoffe, Lösungsmittel, Stabilisatoren, Puffer, den pH-Wert stabilisierende Mittel und/oder Salze.

7. Zubereitung nach einem der Ansprüche 1 bis 6 in Form eines Pulvers, einer Brausetablette, eines Dragees, einer Tablette, einer Lösung in einem physiologisch annehmbaren Lösungsmittel oder einer Paste.

8. Zubereitung nach einem der Ansprüche 1 bis 7 zur oralen Einnahme oder zur intravenösen Verabreichung.

9. Zubereitung nach einem der Ansprüche 1 bis 8 für medizinische Zwecke und/oder für diätetische Zwecke

10. Zubereitung nach einem der Ansprüche 1 bis 8 für ernährungsphysiologische Zwecke, insbesondere als Nahrungsergänzungsmittel.

## Claims

1. Composition containing glucose and/or one or more compound(s) releasing glucose under physiological conditions and chromium in a resorbable form as well as well an emulsifying agent/active agent together with physiologically acceptable carriers and auxiliary agents, wherein
- glucose and/or one or more compound(s) releasing glucose under physiological conditions is/are contained in a concentration of 30 to 60 % by weight;
- chromium is contained in a concentration of 0.002 to 0.01 % by weight; and
- one or more emulsifying agent(s)/active agent(s) is/are contained in a total concentration of 0.5 to 2 % by weight.

2. Composition according to claim 1, containing choline bitartrate, instead of or together with one or more emulsifying agent(s), in a concentration of 0.05 to 0.5 % by weight, preferably in a concentration of 0.1 to 0.3 % by weight, particularly preferably in a concentration 0.15 to 0.2 % by weight.

3. Composition according to claim 1 or claim 2, wherein glucose and/or one or more compound(s) releasing glucose under physiological conditions is/are contained in a concentration of 35 to 50 % by weight, preferably wherein, as glucose and compound(s) releasing glucose under physiological conditions, D-glucose, L-glucose, D-glucose or L-glucose containing disaccharides as saccharose, maltose or lactose, D- or L-glucose containing polysaccharides as starch or glycogen and mixtures of said compounds is/are contained in a concentration of 35 to 50 % by weight.

4. Composition according to one or more of claims 1 to 3, wherein chromium is contained in a concentration of 0.004 to 0.008 % by weight, preferably wherein chromium, in the form of chromium (III) picolinate, chromium yeast and/or brewer's yeast heaving a natural chromium content is/are contained in a concentration of 0.004 to 0.008 % by weight.

5. Composition according to any of the claims 1 to 4, wherein one or more emulsifying agent(s) is/are contained in a total concentration of 0.8 to 1.8 % by weight, preferably wherein lecithine as an emulsifying agent is contained in a concentration of 0.8 to 1.8 % by weight, even more preferred wherein lecithine from soylecithines, particularly such having a high phosphatidyl content, is contained as emulsifying agent in a concentration of from 0.8 to 1.8 %.

6. Composition according any of the claims 1 to 5, further containing vitamines and trace elements in physiologically acceptable concentrations as well as, optionally instead or in addition, auxiliary agents as flavours, solvents, stabilisors, puffers, agents stabilising the pH-value and/or salts.

7. Composition according any of the claims 1 to 6 in the form of a powder, and effervescent tablet, a coated tablet, a tablet, a solution in a physiologically acceptable solvent or a paste.

8. Composition according any of the claims 1 to 7 for oral administration or intravenous administrations.

9. Composition according any of the claims 1 to 8 for medical purposes and/or for dietetic purposes.

10. Composition according any of the claims 1 to 8 for nutrition-physiological purposes, in particular as food supplement.

## Revendications

1. Préparation contenant du glucose et/ou un ou plusieurs composés qui, dans des conditions physiologiques cèdent du glucose, ainsi que du chrome sous une forme résorbable, ainsi qu'un émulsifiant/matière active, en plus de supports et adjuvants acceptables d'un point de vue physiologique, dans laquelle
- le glucose et/ou un ou plusieurs composés qui dans des conditions physiologiques cèdent le glucose sont présents à une concentration de 30 à 60 % en poids ;
- le chrome est présent à une concentration de 0,002 à 0.01 % en poids ; et
- un ou plusieurs émulsifiants/matières actives sont présents à une concentration totale de 0,5 à 2 % en poids.

2. préparation selon la revendication 1, contenant, au lieu d'un ou plusieurs émulsifiants, ou en même temps que ces derniers, du bitartrate de choline à une concentration de 0,05 à 0,5 % en poids, de préférence à une concentration de 0,1 à 0,3 et plus particulièrement de 0,15 à 0,2 % en poids.

3. Préparation selon la revendication 1 ou 2, dans laquelle le glucose et/ou un ou plusieurs composés qui dans des conditions physiologiques cèdent le glucose sont présents à une concentration de 35 à 50 % en poids, de préférence dans laquelle on a, présents à une concentration de 35 à 50 % en poids, en tant que glucose et composés qui dans des conditions physiologiques cèdent le glucose, du D-glucose, du L-glucose, des disaccharides contenant du D- ou du L-glucose tels que le saccharose, le maltose ou le lactose, des polysaccharides contenant du D- ou du L-glucose tels que l'amidon ou le glycogène, et des mélanges des composés mentionnés.

4. Préparation selon l'une ou plusieurs des revendications 1 à 3, dans laquelle le chrome est présent à une concentration de 0,004 à 0,008 % en poids, de préférence dans laquelle le chrome est présent à une concentration de 0,004 à 0,008 % en poids sous forme de picolinate de chrome(III), de levure de chrome et/ou de levure de bière contenant naturellement du chrome.

5. Préparation selon l'une des revendications 1 à 4, dans laquelle on a présents un ou plusieurs émulsifiants à une concentration totale de 0,8 à 1,2 % en poids, de préférence dans laquelle de la lécithine est présente en tant qu'émulsifiant à une concentration de 0,8 à 1,8 % en poids, et plus particulièrement dans laquelle on a présente, en tant qu'émulsifiant à une concentration de 0,8 à 1,8 % en poids, de la lécithine choisie parmi les lécithines du soja, en particulier celles qui ont une proportion élevée de phosphatidyle.

6. Préparation selon l'une des revendications 1 à 5, qui contient en outre des vitamines et des oligo-éléments à des concentrations acceptables d'un point de vue physiologique, et éventuellement, à la place ou en plus de ces derniers, des adjuvants tels que des agents de sapidité, des solvants, des stabilisants, des tampons, des agents stabilisant le pH et/ou des sels.

7. Préparation selon l'une des revendications 1 à 6 sous forme d'une poudre, d'un comprimé effervescent, d'une dragée, d'un comprimé, d'une solution dans un solvant acceptable d'un point de vue physiologique, ou d'une pâte.

8. Préparation selon l'une des revendications 1 à 7, pour prise orale ou pour administration intraveineuse.

9. Préparation selon l'une de revendications 1 à 8, à des fins médicinales et/ou à des fins diététiques.

10. Préparation selon l'une des revendications 1 à 8, à des fins de physiologie de la nutrition, en particulier en tant que complément alimentaire.
